# EUROPEAN PATENT SPECIFICATION

(11) **EP 1 622 651 B1**
(45) Date of publication and mention of the grant of the patent: **14.11.2012**
(21) Application number: 03752144.0
(22) Date of filing: 03.09.2003
(51) Int. Cl.: A23L 3/28, A61L 2/10, C02F 1/32, A61L 2/26

(54) **MODULAR, HIGH VOLUME, HIGH PRESSURE LIQUID DISINFECTION USING UV RADIATION**
MODULARE, HOCHVOLUMIGE HOCHDRUCK-FLÜSSIGDESINFEKTION MIT UV-STRAHLUNG
DISPOSITIF MODULAIRE POUR DESINFECTER UN GRAND VOLUME DE LIQUIDE SOUS HAUTE PRESSION A L'AIDE D'UN RAYONNEMENT UV

(30) Priority: 21.01.2003 US 441930 P
(43) Date of publication of application: 08.02.2006
(73) Proprietor: Safe Foods Corporation, North Little Rock, AR 72118 (US)
(72) Inventor: NOLEN, Gary, Bellavista, AR 72715 (US); RHEINGANS, Joe, Rogers, AR 72756 (US)
(74) Representative: Trinks, Ole
(86) International application number: PCT/US2003/028218
(87) International publication number: WO 2004/067048

(56) References cited:
- EP-A- 0 582 739
- DE-A1- 4 233 566
- US-A- 4 968 891
- US-A- 5 178 758
- US-A- 5 597 482
- US-A- 5 997 812
- US-A- 5 997 812
- US-A1- 2002 096 648

## Description

### Background of the Invention

The present invention relates to liquid disinfection and, more particularly, to liquid disinfection using ultraviolet (UV) radiation.

U.S. Patent No. 5,997,812 discloses applications and methods for disinfecting contaminated fluids using a high-gauss magnet followed by ultraviolet radiation. In an embodiment ultraviolet lamps and associated reflectors are disposed around a UV-transmissible tube.

EP 0 582 739 discloses an apparatus for irradiating cells passing through hollow tubing which is helically wrapped around an outer cylinder. A UV light source is disposed within an inner cylinder which is arranged within the outer cylinder. For ventilation and temperature control, air may be blown into the inner cylinder 26, through apertures, and through the space between the inner and the outer cylinder.

It is known to use UV radiation to disinfect clear or opaque liquids such as water, including wastewater, juices, brines, marinades, beverage, and the like. A couple of examples include U.S. Patent Nos. 3,527,940 and 4,968,891. Using UV radiation to disinfect liquids offers many advantages that often make it a very attractive option as compared to other methods of disinfecting liquids. It will often provide for improved disinfection in a fast, simple, relatively inexpensive manner,

Still, prior equipment and methods of disinfecting liquids using UV radiation suffer from a number of disadvantages. For example, the relatively fragile nature of the equipment has placed undesirable limitations on the flow rates that may be treated and operating pressures that may be used. The relatively fragile nature of the equipment similarly limited pressures and flow rates that could be used for cleaning purposes, making it more difficult or impossible to provide the convenience of clean in place equipment. The effectiveness of UV radiation to disinfect a liquid diminishes rapidly, likely exponentially, with distance, so relying primarily upon turbulence in a liquid to provide for even, thorough disinfection of the liquid can be unreliable. Also, exposure times for desired levels of disinfection can often lead to the use of undesirably large equipment or the use of an undesirably large number of units of such equipment, adding to the cost of the system and taking up valuable floor space, In a typical prior art unit, a significant portion of the radiation emitted by the bulbs is not directed toward the liquid to be treated and is wasted, making inefficient use of the radiation and of the power consumed to generate the radiation. Prior cabinets or units used to provide UV disinfection of liquids also provided little or no flexibility in handling differing flow patterns, flow rates, and treatment times. Further, prior cabinets and units were difficult and time-consuming to service or repair, and typically required an entire cabinet or unit to be shut down and placed out of service for extended periods.

### Summary of the Invention

It is therefore an object of the present invention to provide a system and method for treating a liquid with radiation that offers increased efficiency.

It is a further object of the present invention to provide a system of the above type that allows the flexibility of switching between parallel and series flow with minimal adjustments.

It is a still further object of the present invention to provide a system of the above type that provides a rugged system that may handle high pressures and flow rates.

It is a still further object of the present invention to provide a system of the above type which uses modular illumination units to allow for fast and easy replacement of bulbs or other components.

It is a still further object of the present invention to provide a system of the above type that makes highly efficient use of radiation generated by treating bulbs.

It is a still further object of the present invention to provide a system of the above type which provides for an extended treatment path without a corresponding increase in the length of the treatment chamber.

It is a still further object of the present invention to provide a system of the above type which provides for more even and thorough exposure of the liquid to be treated.

It is a still further object of the present invention to provide a system of the above type which provides for the convenience of fluid input and output at the same end of a treatment chamber.

Toward the fulfillment of these and other objects and advantages, an apparatus according to claim 1 and a method according to claim 9 are provided.

### Brief Description of the Drawings

The above brief description, as well as further objects, features and advantages of the present invention will be more fully appreciated by reference to the following detailed description of the presently preferred but nonetheless illustrative embodiments in accordance with the present invention when taken in conjunction with the accompanying drawings, wherein:
FIG. 1 is a sectional, side elevation view of a treatment chamber forming part of a radiation treatment device of the present invention;
FIG. 2 is a sectional, overhead view of a radiation treatment device of the present invention;
FIG. 3 is a partial, side elevation view of an alternate embodiment of a radiation treatment device of the present invention;
FIG. 4 is a is a partial, sectional, overhead view of an alternate embodiment of a radiation treatment device of the present invention;
FIG. 5 is an overhead, perspective view of a parallel flow alignment of a radiation treatment device of the present invention;
FIG. 6 is an overhead, perspective view of a series flow alignment of a radiation treatment device of the present invention;
FIG. 7 is a front elevation view of a cabinet for housing a radiation treatment device of the present invention; and
FIG. 8 is a partial, side elevation view of a cabinet for housing a radiation treatment device of the present invention.

### Detailed Description of the Referred Embodiment

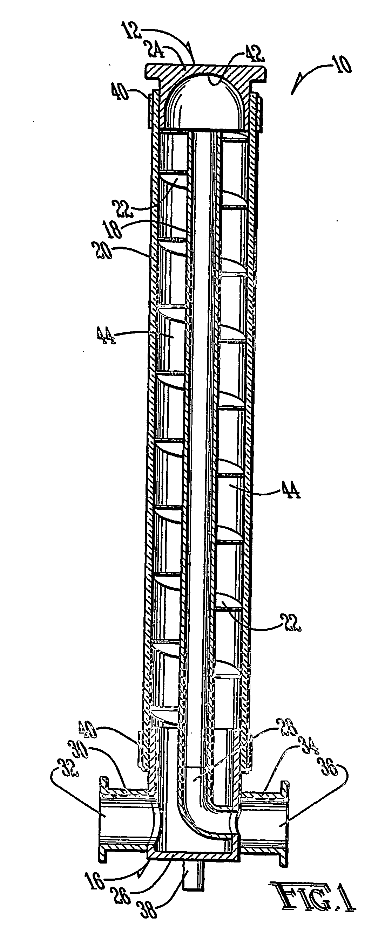

Referring to Fig. 1, the reference numeral 10 refers in general to a radiation treatment device of the above invention. The device 10 comprises a treatment chamber 12 and a radiation source 14 disposed in close proximity thereto.

The treatment chamber 12 comprises a header 16, inner and outer tubes 18 and 20, a static mixer 22, and an end cap 24. The header 16 has an outer housing 26, an inner header tube 28, an input pipe 30 with an input opening 32, and an output pipe 34 with an output opening 36. The outer housing 26 is open at the top, closed at the bottom, and has two side openings disposed on opposite sides, with one side opening being larger than the other. A mount 38 is secured to the bottom wall of the outer housing 26. The input pipe is affixed to the outer housing 26, aligned with the larger of the two side openings. The output pipe 34 is affixed to the outer housing 26 aligned with the smaller of the two other side openings. The input and output pipes 30 and 34 both have inner diameters of approximately 38,1mm (1.5 inches). The inner diameter of the output pipe 34 is larger than the diameter of the side opening. The inner header tube 28 has an input opening centrally disposed and coaxially aligned with the outer housing 26 and an output opening aligned with the smaller of the two side openings. The inner diameter of the inner header tube 28 is substantially the same as the diameter of this side opening. The header 16 is preferably made of stainless steel and is of clean in place construction. It is of course understood that the header 16 may be made of any number of different materials or combinations of materials. It is also understood that the header 16 may be assembled or fabricated from a number of different parts or may be cast or molded as one or more integral pieces.

Outer tube 20 is made of a material that is transparent to UV radiation or to the type of radiation used. The outer tube 20 is preferably constructed of a polymer, is more preferably constructed of a fluoropolymer, and is most preferably constructed of fluorinated ethylene propylene. The outer tube may of course be constructed of any number of materials known to possess the desired degree of transparency. The outer tube 20 has a length of approximately 1,52 m (60 inches) and has an inner diameter of approximately 31,8 mm(1.25 inche). A lower portion of the outer tube 20 is secured to the header 16, such as by using a hose clamp 40. The end cap 24 is affixed to an upper portion of the outer tube 20, such as by using a hose clamp 40. A lower surface 42 of the end cap 24 is curved to assist in redirection of the liquid with minimal pressure drop. The cap 24 is preferably stainless steel.

An output end of the inner tube 18 is affixed to the input end of the inner header tube 28, and the inner tube 18 extends coaxially aligned within the outer tube 20 along most if not all of the height of the outer tube 20. The inner tube 18 is preferably, stainless steel having an inner diameter of substantially within a range of from approximately 12,7 mm (3.5 inch) to approximately 82,6 mm (3.25 inch). The inner tube has an outer diameter that is substantially within a range of approximately from approximately 19,1 mm (0.75 inch) to approximately 88,9 m (3.5 inch). The outer diameter of the inner tube 18 and the inner diameter of the outer tube 20 are preferably selected to provide a relatively narrow annulus 44 between the two having a width of approximately 6,35 mm (0.25 inch). An inner surface of the inner tube 18 defines an inner flow path. An inner surface of outer tube 20 and an outer surface of inner tube 18 define an outer flow path. An opening in a distal end of the inner tube 18 places the outer flow path in fluid flow communication with the inner flow path. The outer surface of the inner tube 18 is not transparent with respect to the radiation from the radiation source 14 and is preferably reflective of the radiation.

The static mixer or helical member 22 is an auger style static mixer that is affixed to the outer diameter of the inner tube 18, such as by welding. The mixer 22 extends between the outer wall of the inner tube 18 and the inner wall of the outer tube 20 and preferably contacts the inner wall of the outer tube 20. The mixer 22 is preferably stainless steel. Different degrees of winding may be used depending upon desired characteristics of the device 10. In one preferred embodiment the winding provides a liquid travel path of approximately 99,1 mm (3.9 inches) for each 25,4 mm inch) of annulus 44 height. For a treatment chamber 12 in which the height of the annulus 44 area is approximately 1,52 m (60 inches) this would provide a liquid travel path of approximately 5,9 m (234 inches).

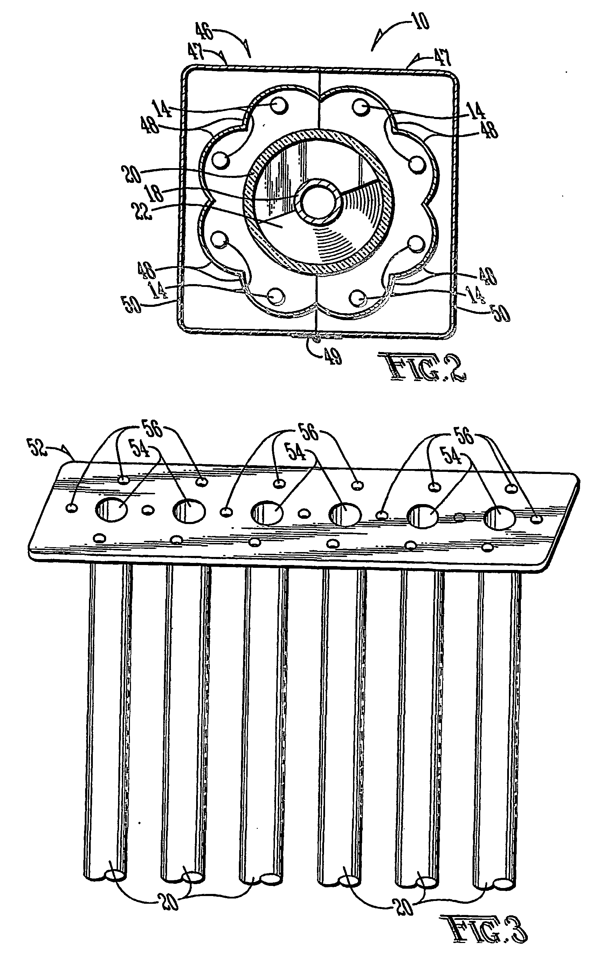

Referring to Fig. 2, a modular illumination unit 46 is provided, formed from two mirror image sections 47. The sections 47 are connected to one another by a hinge 49 or in any conventional manner. Each section 47 comprises a plurality of bulbs 14, one or more reflectors 48, and a bracket 50. The bracket 50 supports and aligns the bulbs 14 and support and aligns the reflector or reflectors 48 positioned adjacent to the bulbs 14, The reflector 48 is configured with a curved portion or segment, such as a semi-circular, hyperbolic, or parabolic shaped portion or segment, associated with each bulb 14, disposed and aligned to reflect and focus radiation emitted from outer portions of the bulb 14 back toward the treatment chamber 12. The segments are disposed so that the reflector 48 is generally clamshell shaped. In that regard, a cross section of one segment falling in a common plane of a cross section of an adjoining segment does not form a portion of a common circle or semicircle with the cross section of the adjoining segment. Each cross section is preferably semi-circular, and each cross section of a segment has an arc length that is greater than approximately 45°. The inner surface of the reflector 48 is selected to be highly reflective of the radiation used. For example, if a UV bulb 14 is used, the inner surface is preferably polished aluminum. Each section 47 is secured to its mating section 47 and is secured within the cabinet 66 in any number of ways, such as being secured to a back wall of the cabinet or to brackets disposed within the cabinet 66. In the preferred embodiment, one section 47 is disposed toward a back portion of the cabinet 66, and a mating section 47 is disposed toward a front portion of the cabinet 66 so that the front section 47 may be easily opened to provide access to the treatment chamber12 and to the sections 47 of the illumination unit 46. Each section 47 is independently removable without the need to remove an associated treatment chamber or mated section 47. The brackets 50 of each section 47 are disposed to place the bulbs 14 in very close proximity to the outer surface of the outer tube 20. In the preferred embodiment, in which the modular concept is used, a separate modular illumination unit 46 is associated with each treatment chamber 12. It is also preferred to provide an extra or spare modular illumination unit 46 along with the device 10. This will reduce down time by making it easy to quickly replace an installed unit 46 with a spare unit 46 if the installed unit is in need of repair, maintenance, or replacement.

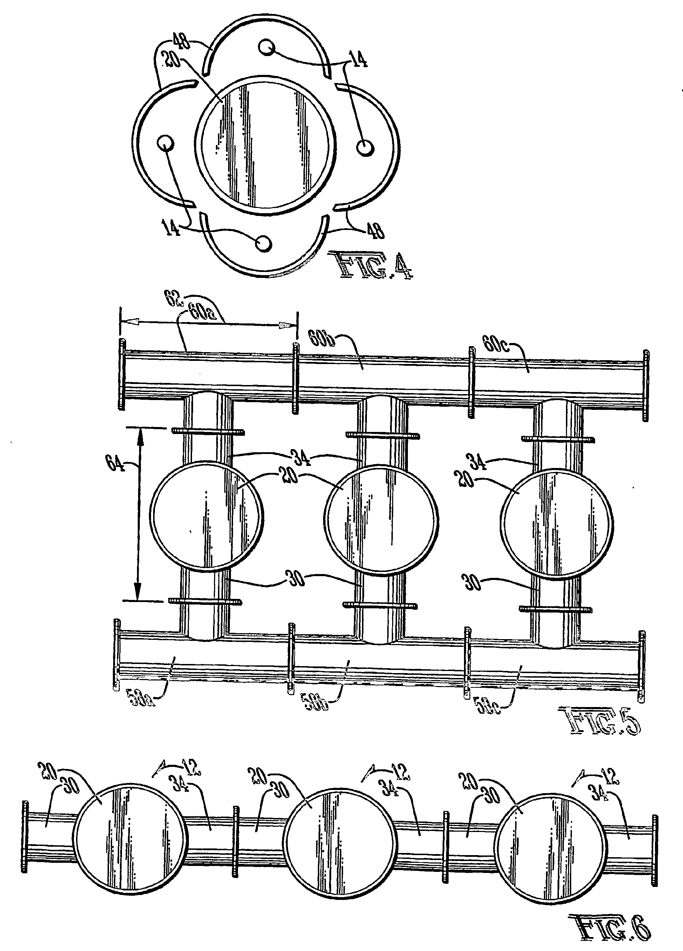

In an alternate embodiment depicted in Figs. 3 and 4, one or more bulb racks 52 may be used to support and align a plurality of outer tubes 20 of a plurality of treatment chambers 12, along with the bulbs 14 and reflectors 48 to be used with each treatment device 10. As seen in Fig. 3, sets of holes or openings 54 and 56 are provided to support and align the outer tubes 20 and bulbs 14, respectively.

Referring to Fig. 5, input and output manifolds 58 and 60 are provided and are disposed to allow for parallel flow of a liquid through a plurality of adjacent treatment chambers 12. The manifolds are provided in a modular arrangement with a first set of associated input and output manifold segments 58a and 60a, a second set of associated input and output manifold segments 58b and 60b, and so on for the desired number of treatment chambers 12 to be used. The length 62 of the each input and output manifold 58, 60 segment is equal to the distance 64 between the input opening 32 of the input pipe 30 and the output opening 36 of the output pipe 34. This allows each treatment chamber 12 to be quickly and easily adjusted to provide for either parallel flow as seen in Fig. 5 or to provide for series flow as seen in Fig. 6.

Fig. 6 shows a plurality of treatment chambers 12 arranged to provide for series flow through a plurality of treatment chambers 12. In this arrangement, the output opening 36 of an output pipe 34 of a first treatment chamber 12 is aligned with an input opening 32 of an input pipe 30 of a second treatment chamber 12, and so on for the desired number of treatment chambers 12.

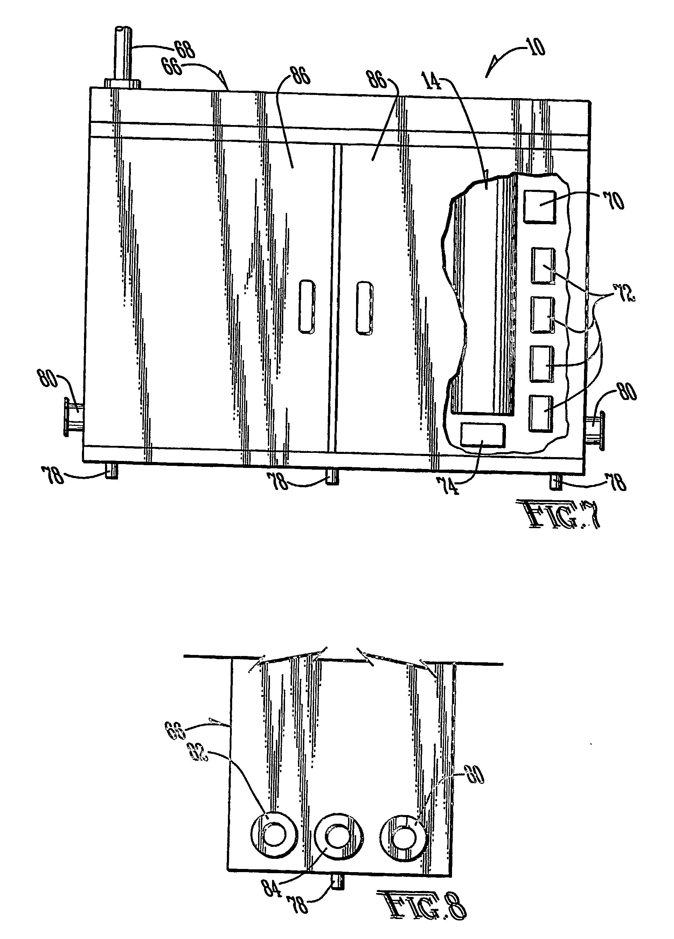

As shown in Fig. 7, a radiation treatment device 10 of the present invention may also include a cabinet 66 and related components. One or more treatment chambers 12 and sets of associated bulbs 14, reflectors 48, and input and output manifolds 58, 60 are housed within the cabinet 66. The cabinet 66 is preferably made primarily of stainless steel. Other components may be disposed within or positioned near the cabinet 66. For example, a power line 68 may supply power to controls 70 and to ballast 72 associated with each bulb 14, which may be housed in the cabinet 66 or separately above the cabinet 66. A fan 74 may be provided for cooling the ballast 72 and controls 70, and drain pipes 78 may be provided in the cabinet 66 floor. In the preferred embodiment, a separate fan 74 will be associated each modular illumination unit 46, with the fan 74 disposed to provide a positive pressure cabinet. It is of course understood that any number of different fan 74 arrangements may be used and that one or more fans may be disposed to provide either a positive pressure cabinet or a negative pressure cabinet. One or more input or output pipes 80, 82, and 84 may be provided, disposed in lower side walls of the cabinet 66. As best seen in Fig. 8, outer pipes 80 and 82 are disposed to align with input and output manifolds 58 and 60, respectively, to provide a path for parallel flow of liquid through the treatment chambers 12 such as when the treatment chambers 12 are aligned as depicted in Fig. 5 . The centrally located pipes 84 are disposed to align with input and output pipes 30 and 34, of the treatment chambers 12 when the treatment chambers 12 are aligned for series flow, such as seen in Fig. 6.

Referring to Figs. 5 and 6, in operation, a plurality of treatment chambers 12 are aligned as desired to provide for parallel or series flow through the desired number of treatment chambers 12. It is of course understood that a single treatment chamber 12 may also be used if desired. Once the treatment chambers 12 are aligned as desired and the cabinet doors 86 closed for added protection against exposure to UV radiation, the bulbs 14 are activated to provide UV radiation. The liquid to be treated is then provided to the device 10 at the desired pressure and flow rate. It is understood that the device 10 may be used in connection with almost liquid, including but not limited to clear or opaque liquids such as water, including wastewater, juices, brines, marinades, beverage, and the like.

In parallel flow (Fig, 5) the liquid will pass through and fill the desired number of input manifold segments 58a, 58b, 58c and will pass from each input manifold 58 segment into an associated treatment chamber 12. As best seen in Fig. 1, the liquid passes through the input pipe 30, through the housing 26, and into the annulus 44 between the inner tube 18 and outer tube 20. The static mixer 22 routes the liquid in a tight spiral pattern along a helical path upward through the annulus 44 to an upper portion of the treatment chamber 12. As the liquid passes through the narrow annulus 44 in close proximity to the bulbs 14, UV radiation from the bulbs 14 provides the desired degree of disinfection. The use of the auger style static mixer 22 provides for significant mixing and churning of the liquid as it passes upward through the annulus 44 so that different portions of the liquid are constantly being moved closer to and further from the bulbs 14. This ensures thorough and even radiation exposure throughout the liquid and greatly reduces the chances of leaving isolated portions relatively untreated or significantly over-treated. The end cap 24 arrests upward flow of the liquid and redirects the liquid to flow downward through the inner tube 18. The liquid then passes through the inner tube 18, through the inner header tube 28, and through the output pipe 34. If the treatment chamber 12 is aligned to provide for parallel flow (Fig. 5), the liquid passes from the output pipe 34 to and through the associated output manifold 60 segment for further use or treatment. If the treatment chamber 12 is aligned to provide for series flow (Fig. 6), the liquid passes from the output pipe 34 of one treatment chamber 12 to the input pipe 30 of another treatment chamber 12 to repeat the process described above.

The rugged device 10 of the present invention may be operated under wide ranges or pressures and flow rates without fear of damaging the device 10. For example, the device 10 of the present invention may be safely operated at a working pressure reaching or exceeding a pressure that is referably substantially within a range of from approximately 206,8 KPa (30 psig) to approximately 413,7 KPa (60 psig) and that is more preferably approximately 393 KPa (57 psig). The device 10 may withstand burst pressures reaching or exceeding a pressure that is preferably substantially within a range of from approximately 689,5 KPa (100 psig) to approximately 2068,4 KPa (300 psig) and that is more preferably approximately 1971,9 KPa (286 psig). Desired flow rates for many applications will typically be within a range of from approximately 1 gallon per minute to approximately 75,7 l (20 gallons) per minute. Similarly, desired flow rates for typical clean in place cleaning will typically be less than or equal to approximately 94,9 l (25 gallons) per minute. Still, much higher How rates may be desirable for some applications, such as for the batch processing of juice. In the batch processing of juice, it is sometimes desirable to process flow rates reaching or exceeding approximately 265 l (70 gallons) per minute. Because of limitations imposed by the relatively fragile nature of prior radiation treatment devices, it is not believed that UV radiation treatment has been used in applications calling for such high flow rates. In contrast, the rigid construction of the present invention will preferably allow the present invention to safely process flows rates of up to approximately 113,6 l (30 gallongs) per minute, will more preferably allow the present invention to safely process flows rates of up to approximately 208,2 l (55 gallons) per minute, and will most preferably allow the present invention to safely process flows rates of up to approximately 302,82 l (80 gallons) per minute. A treatment chamber 12 typically processes approximately 37,9 to 45,4 l(10 to 12 gallons) per minute. Parallel flow is typically used for higher rates.

Other modifications, changes and substitutions are intended in the foregoing, and in some instances, some features of the invention will be employed without a corresponding use of other features. For example, any number of treatment chambers 12 may be used, from one to several. Similarly, although it is preferred to use a configuration of eight bulbs 14 per treatment chamber 12, any number of bulbs 14 may be used in connection with a treatment chamber 12, from one to several. Also, any number of different types of mixers 22 may be used in the annulus 44, or a mixer 22 may be omitted. Further, any number of different flow paths may be used, including but not limited to a flow path that is roughly the reverse of that described in the preferred embodiment. Similarly, strictly series flow may be used, strictly parallel flow may be used, or any number of combinations of series and parallel flows may be used. Also, the header 16 may be disposed in different locations, such as at the top of the treatment chamber 12. Similarly, any number of different methods may be used to route the fluid to or from the annulus 44 area and to or from the inner tube 18. Although bulbs 14 providing UV radiation are preferred, any number of different types of radiation and types of radiation sources 14 may be used depending upon the desired application. Further, the reflectors 48 may take any number of shapes, sizes or configurations or may be omitted, Further still, any number of different structures and arrangements may be used for supporting and aligning the various components of the device. Similarly, any number of different structures and arrangements may be provided for shielding users and surrounding environments from radiation exposure. Although the preferred embodiment, is particularly useful for treating liquids, it is of course understood that the invention may be used in connection with treating any number of different forms of matter. For example, a device of the present invention may also be used to treat a gas or to treat fluid matter, including but not limited to solid particulate mater. It is of course understood that all quantitative information is given by way of example only and is not intended to limit the scope of the present invention.

## Claims

1. An apparatus, comprising:
an outer tube (20) having an inner surface and an outer surface;
an inner tube (18) having an inner surface and an outer surface,
said inner tube (18) being disposed within said outer tube (20) so that said inner surface of said outer tube and said outer surface of said inner tube define an outer flow path and so that said inner surface of said inner tube defines an inner flow path,
said inner tube having an opening, said opening placing said outer flow path in fluid flow communication with said inner flow path;
a radiation source (14) being disposed adjacent to said outer surface of said outer tube (20),
**characterized by**
a header (16) being affixed to a proximal end portion of said outer tube (20) and to a proximal end portion of said inner tube (18), said header being in fluid flow communication with said outer flow path and said inner flow path,
said outer and said inner flow paths being flow paths for liquid to be radiated.

2. The apparatus of claim 1, further comprising: a helical member (22) disposed within said outer flow path.

3. The apparatus of claim 1, wherein said header (16) defines an input flow path and a separate output flow path, said input flow path being in fluid flow communication with said outer flow path or said inner flow path and said output flow path being in fluid flow communication with the other of said outer flow path or said inner flow path.

4. The apparatus of claim 1, wherein said header (16) defines an input flow path and a separate output flow path, said input flow path being in fluid flow communication with said outer flow path and said output flow path being in fluid flow communication with said inner flow path.

5. The apparatus of claim 1, further comprising: an end cap (24) affixed to a distal end portion of said outer tube (20).

6. The apparatus of claim 5, wherein said opening of said inner tube (18) is disposed at the distal end portion of said inner tube.

7. The apparatus of claim 1, wherein said inner tube (18) is not transparent with respect to radiation from said radiation source (14).

8. The apparatus of claim 1, wherein said inner tube (18) is comprised of stainless steel.

9. A method, comprising:
(1) providing a first tube (20) and a second tube (18), said second tube (18) being disposed at least partially within said first tube (20), an inner wall of the first tube (20) and an outer wall of the second tube (18) forming an outer flow path, an interior of said second tube (18) forming an inner flow path, a header (16) being affixed to a proximal end portion of said first tube (20) and to a proximal end portion of said second tube (18), said header being in fluid flow communication with said outer flow path and said inner flow path,
(2) passing a liquid between said inner wall of said first tube (20) and said outer wall of said second tube (18) forming said first flow path;
(3) irradiating said liquid as said liquid passes between said inner wall of said first tube and said outer wall of said second tube,
(4) before or after step (2), passing said liquid through said interior of said second tube (18) forming the second flow path.

10. The method of claim 9, wherein step (4) comprises: after step (2), passing said liquid through said interior of said second tube (18).

11. The method of claim 9, wherein step (2) comprises: passing said liquid along a helical path between said inner wall of said first tube (20) and said outer wall of said second tube (18).

12. The method of claim 11, wherein (3) comprises: irradiating said liquid with a UV bulb (14) as said liquid passes between said inner wall of said first tube (20) and said outer wall of said second tube (18).

13. The method of claim 9, wherein step (2) comprises: passing said liquid between said inner wall of said first tube (20) and said outer wall of said second tube (18) at a pressure that is greater than or equal to approximately 206,8 kPa (30 psig).

14. The method of claim 9, wherein step (2) comprises: passing said liquid between said inner wall of said first tube (20) and said outer wall of said second tube (18) at a flow rate that is greater than or equal to approximately 37,9 I (10 gallons) per minute.

## Patentansprüche

1. Gerät, das Folgendes umfasst:
einen Außenschlauch (20) mit einer Innenseite und einer Außenseite;
einen Innenschlauch (18) mit einer Innenseite und einer Außenseite, wobei der Innenschlauch (18) im Außenschlauch (20) angeordnet ist, so dass die Innenseite des Außenschlauchs und die Außenseite des Innenschlauchs einen äußeren Strömungspfad definieren und so dass die Innenseite des Innenschlauchs einen inneren Strömungspfad definiert,
wobei der Innenschlauch eine Öffnung aufweist, die den äußeren Strömungspfad mit dem inneren Strömungspfad in Flüssigkeitsströmungsverbindung bringt;
eine neben der Außenseite des Außenschlauchs (20) angeordnete Strahlenquelle (14),
**gekennzeichnet durch**
ein Kopfstück (16), das an einem proximalen Endabschnitt des Außenschlauchs (20) und an einem proximalen Endabschnitt des Innenschlauchs (18) befestigt ist, wobei das Kopfstück mit dem äußeren Strömungspfad und dem inneren Strömungspfad in Flüssigkeitsströmungsverbindung steht,
die äußeren und inneren Strömungspfade für zu bestrahlende Flüssigkeit sind.

2. Gerät nach Anspruch 1, das ferner Folgendes umfasst: ein im äußeren Strömungspfad angeordnetes spiralförmiges Glied (22).

3. Gerät nach Anspruch 1, worin das Kopfstück (16) einen Eingangsströmungspfad und einen separaten Ausgangsströmungspfad definiert, wobei der Eingangsströmungspfad mit dem äußeren Strömungspfad oder dem inneren Strömungspfad in Flüssigkeitsströmungsverbindung steht und der Ausgangsströmungspfad mit dem jeweils anderen der äußeren oder inneren Strömungspfade in Flüssigkeitsströmungsverbindung steht.

4. Gerät nach Anspruch 1, worin das Kopfstück (16) einen Eingangsströmungspfad und einen separaten Ausgangsströmungspfad definiert, wobei der Eingangsströmungspfad mit dem äußeren Strömungspfad in Flüssigkeitsströmungsverbindung steht und der Ausgangsströmungspfad mit dem inneren Strömungspfad in Flüssigkeitsströmungsverbindung steht.

5. Gerät nach Anspruch 1, das ferner Folgendes umfasst: eine an einem distalen Endabschnitt des Außenschlauchs (20) befestigte Endkappe (24).

6. Gerät nach Anspruch 5, worin die Öffnung des Innenschlauchs (18) am distalen Endabschnitt des Innenschlauchs angeordnet ist.

7. Gerät nach Anspruch 1, worin der Innenschlauch (18) gegenüber Strahlung von der Strahlenquelle (14) nicht transparent ist.

8. Gerät nach Anspruch 1, worin der Innenschlauch (18) aus Edelstahl besteht.

9. Verfahren, das Folgendes umfasst:
(1) Bereitstellung eines ersten Schlauchs (20) und eines zweiten Schlauchs (18), wobei der zweite Schlauch (18) zumindest teilweise im ersten Schlauch (20) angeordnet ist, wobei eine Innenwand des ersten Schlauchs (20) und eine Außenwand des zweiten Schlauchs (18) einen äußeren Strömungspfad bilden, eine Innenseite des zweiten Schlauchs (18) einen inneren Strömungspfad bildet, ein Kopfstück (16) an einem proximalen Endabschnitt des ersten Schlauchs (20) und an einem proximalen Endabschnitt des zweiten Schlauchs (18) befestigt ist, wobei das Kopfstück mit dem äußeren Strömungspfad und dem inneren Strömungspfad in Flüssigkeitsströmungsverbindung steht,
(2) Durchleiten einer Flüssigkeit zwischen der Innenwand des ersten Schlauchs (20) und der Außenwand des zweiten Schlauchs (18) zur Bildung des ersten Strömungspfads;
(3) Bestrahlen der Flüssigkeit während ihrer Durchleitung zwischen der Innenwand des ersten Schlauchs und der Außenwand des zweiten Schlauchs,
(4) vor oder nach Schritt (2) Durchleiten der Flüssigkeit durch den Innenraum des zweiten Schlauchs (18) zur Bildung des zweiten Strömungspfads.

10. Verfahren nach Anspruch 9, worin Schritt (4) Folgendes umfasst: nach Schritt (2) Durchleiten der Flüssigkeit durch den Innenraum des zweiten Schlauchs (18).

11. Verfahren nach Anspruch 9, worin Schritt (2) Folgendes umfasst: Durchleiten der Flüssigkeit auf einem spiralförmigen Pfad zwischen der Innenwand des ersten Schlauchs (20) und der Außenwand des zweiten Schlauchs (18).

12. Verfahren nach Anspruch 11, worin (3) Folgendes umfasst: Bestrahlen der Flüssigkeit mit einer UV-Glühbirne (14) während der Durchleitung der Flüssigkeit zwischen der Innenwand des ersten Schlauchs (20) und der Außenwand des zweiten Schlauchs (18).

13. Verfahren nach Anspruch 9, worin Schritt (2) Folgendes umfasst: Durchleiten der Flüssigkeit zwischen der Innenwand des ersten Schlauchs (20) und der Außenwand des zweiten Schlauchs (18) mit einem Druck, der größer gleich 206,8 kPa (30 psig) ist.

14. Verfahren nach Anspruch 9, worin Schritt (2) Folgendes umfasst: Durchleiten der Flüssigkeit zwischen der Innenwand des ersten Schlauchs (20) und der Außenwand des zweiten Schlauchs (18) mit einer Strömungsrate größer gleich ca. 37.9 1 (10 Gallonen) pro Minute.

## Revendications

1. Appareil comportant :
un tube extérieur (20) présentant une surface intérieure et une surface extérieure ;
un tube intérieur (18) présentant une surface intérieure et une surface extérieure, ledit tube intérieur (18) étant disposé à l'intérieur dudit tube extérieur (20) de telle façon que ladite surface intérieure dudit tube extérieur et ladite surface extérieure dudit tube intérieur définissent un passage extérieur d'écoulement et de telle façon que ladite surface intérieure dudit tube intérieur définisse un passage intérieur d'écoulement,
ledit tube intérieur présentant une ouverture, ladite ouverture plaçant ledit passage extérieur d'écoulement en communication d'écoulement de fluide avec ledit passage intérieur d'écoulement ;
une source (14) de rayonnement étant disposée de façon adjacente à ladite surface extérieure dudit tube extérieur (20),
**caractérisé**
**en ce qu'**un collecteur (16) est apposé sur une partie d'extrémité proximale dudit tube extérieur (20) et sur une partie d'extrémité proximale dudit tube intérieur (18), ledit collecteur étant en communication d'écoulement de fluide avec ledit passage extérieur d'écoulement et ledit passage intérieur d'écoulement, lesdits passages extérieur et intérieur d'écoulement étant des passages d'écoulement destinés à un liquide à irradier.

2. Appareil selon la revendication 1, comportant en outre : un organe hélicoïdal (22) disposé à l'intérieur dudit passage extérieur d'écoulement.

3. Appareil selon la revendication 1, ledit collecteur (16) définissant un passage d'écoulement d'entrée et un passage séparé d'écoulement de sortie, ledit passage d'écoulement d'entrée étant en communication d'écoulement de fluide avec ledit passage extérieur d'écoulement ou ledit passage intérieur d'écoulement et ledit passage d'écoulement de sortie étant en communication d'écoulement de fluide avec l'autre desdits passages extérieur et intérieur d'écoulement.

4. Appareil selon la revendication 1, ledit collecteur (16) définissant un passage d'écoulement d'entrée et un passage séparé d'écoulement de sortie, ledit passage d'écoulement d'entrée étant en communication d'écoulement de fluide avec ledit passage extérieur d'écoulement et ledit passage d'écoulement de sortie étant en communication d'écoulement de fluide avec ledit passage intérieur d'écoulement.

5. Appareil selon la revendication 1, comportant en outre : un bouchon (24) d'extrémité apposé sur une partie d'extrémité distale dudit tube extérieur (20).

6. Appareil selon la revendication 5, ladite ouverture dudit tube intérieur (18) étant disposée au niveau de la partie d'extrémité distale dudit tube intérieur.

7. Appareil selon la revendication 1, ledit tube intérieur (18) n'étant pas transparent par rapport au rayonnement provenant de ladite source (14) de rayonnement.

8. Appareil selon la revendication 1, ledit tube intérieur (18) étant constitué d'acier inoxydable.

9. Procédé comportant les étapes consistant à :
(1) mettre en place un premier tube (20) et un deuxième tube (18), ledit deuxième tube (18) étant disposé au moins partiellement à l'intérieur dudit premier tube (20), une paroi intérieure du premier tube (20) et une paroi extérieure du deuxième tube (18) formant un passage extérieur d'écoulement, un intérieur dudit deuxième tube (18) formant un passage intérieur d'écoulement, un collecteur (16) étant apposé sur une partie d'extrémité proximale dudit premier tube (20) et sur une partie d'extrémité proximale dudit deuxième tube (18), ledit collecteur étant en communication d'écoulement de fluide avec ledit passage extérieur d'écoulement et ledit passage intérieur d'écoulement,
(2) faire passer un liquide entre ladite paroi intérieure dudit premier tube (20) et ladite paroi extérieure dudit deuxième tube (18) formant ledit premier passage d'écoulement ;
(3) irradier ledit liquide tandis que ledit liquide passe entre ladite paroi intérieure dudit premier tube et ladite paroi extérieure dudit deuxième tube,
(4) avant ou après l'étape (2), faire passer ledit liquide à travers ledit intérieur dudit deuxième tube (18) formant le deuxième passage d'écoulement.

10. Procédé selon la revendication 9, l'étape (4) comportant une étape consistant : après l'étape (2), à faire passer ledit liquide à travers ledit intérieur dudit deuxième tube (18).

11. Procédé selon la revendication 9, l'étape (2) comportant une étape consistant à : faire passer ledit liquide le long d'un passage hélicoïdal entre ladite paroi intérieure dudit premier tube (20) et ladite paroi extérieure dudit deuxième tube (18).

12. Procédé selon la revendication 11, (3) comportant une étape consistant à : irradier ledit liquide à l'aide d'une ampoule (14) à UV tandis que ledit liquide passe entre ladite paroi intérieure dudit premier tube (20) et ladite paroi extérieure dudit deuxième tube (18).

13. Procédé selon la revendication 9, l'étape (2) comportant une étape consistant à : faire passer ledit liquide entre ladite paroi intérieure dudit premier tube (20) et ladite paroi extérieure dudit deuxième tube (18) à une pression supérieure ou égale à environ 206,8 kPa (30 psi effectifs).

14. Procédé selon la revendication 9, l'étape (2) comportant une étape consistant à : faire passer ledit liquide entre ladite paroi intérieure dudit premier tube (20) et ladite paroi extérieure dudit deuxième tube (18) à un débit supérieur ou égal à environ 37,9 1 (10 gallons) par minute.
